# FASCICULE DE BREVET EUROPEEN

(11) **EP 2 776 596 B1**
(45) Date de publication et mention de la délivrance du brevet: **24.04.2019**
(21) Numéro de dépôt: 12795498.0
(22) Date de dépôt: 07.11.2012
(51) Int. Cl.: C22C 1/02, C22C 14/00, C22F 1/18, C23C 8/24, C23C 14/06, C23C 16/34, A61C 13/00, A61L 27/06, A61L 29/02, A61L 31/02

(54) **PROCÉDÉ DE FABRICATION D'UN ALLIAGE A BASE DE TITANE POUR DISPOSITIFS BIOMEDICAUX**
VERFAHREN ZUR HERSTELLUNG EINER TITANLEGIERUNG FÜR BIOMEDIZINISCHE VORRICHTUNGEN
METHOD FOR MANUFACTURING A TITANIUM ALLOY FOR BIOMEDICAL DEVICES

(30) Priorité: 10.11.2011 FR 1160281
(43) Date de publication de la demande: 17.09.2014
(73) Titulaire: Institut National des Sciences Appliquees de Rennes (INSA de Rennes), 35700 Rennes (FR); Centre National de la Recherche Scientifique (C.N.R.S.), 75016 Paris (FR)
(72) Inventeur: GLORIANT, Thierry, F-35250 Mouaze (FR); GORDIN, Doina, F-35250 Mouaze (FR)
(74) Mandataire: Ipside
(86) Numéro de dépôt international: PCT/FR2012/052572
(87) Numéro de publication internationale: WO 2013/068691

(56) Documents cités:
- EP-A1- 0 449 793
- EP-A1- 0 601 804
- FR-A1- 2 848 810
- LIU X ET AL: "Surface modification of titanium, titanium alloys, and related materials for biomedical applications", MATERIALS SCIENCE AND ENGINEERING: R: REP, ELSEVIER, AMSTERDAM, NL, vol. 47, no. 3-4, 24 December 2004 (2004-12-24), pages 49-121, XP004722113, ISSN: 0927-796X, DOI: 10.1016/J.MSER.2004.11.001
- NAKAI M ET AL: "Surface hardening of biomedical Ti-29Nb-13Ta-4.6Zr and Ti-6Al-4V ELI by gas nitriding", MATERIALS SCIENCE AND ENGINEERING: A, ELSEVIER, AMSTERDAM, NL, vol. 486, no. 1-2, 15 July 2008 (2008-07-15), pages 193-201, XP022633354, ISSN: 0921-5093, DOI: 10.1016/J.MSEA.2007.08.065 [retrieved on 2008-04-29]

## Description

La présente invention concerne un procédé pour fabriquer un alliage à base de titane, présentant des propriétés super élastique et/ou à mémoire de forme, et destiné à constituer des dispositifs utilisés dans le domaine biomédical, tel que par exemple des limes endodontiques, des arcs, fils et autres ressorts orthodontiques, des implants dentaires, des stents cardiovasculaires ou pulmonaires, des fils guide et des cathéters pour la chirurgie cardiovasculaire, des agrafes et prothèses d'articulation pour la chirurgie orthopédique.

Dans le domaine biomédical, les dispositifs et éléments, tels que ceux mentionnés ci-dessus à titre d'exemple, doivent présenter des caractéristiques très différentes, d'une part sur le plan mécanique compte tenu de l'objectif visé consistant à agir sur une partie du corps humain (telle qu'une dent, par exemple dans le cadre d'une application orthodontique), et d'autre part sur le plan biologique, pour éviter, ou atténuer au maximum, les réactions ou conséquences liées au contact du dispositif avec la partie du corps humain ou l'organe.

Parmi les caractéristiques techniques recherchées, voire nécessaires, on peut citer : un domaine d'élasticité recouvrable le plus large possible (propriété de super élasticité), une faible rigidité, une excellente biocompatibilité chimique, une grande résistance à la corrosion et aux produits de stérilisation, une facilité d'usinage et de travail à froid, ainsi qu'une augmentation de la dureté et de la résistance à l'usure superficielle.

De manière connue, on a tenté de concilier ces contraintes qui sont globalement contradictoires les unes avec les autres.

Les alliages super élastiques et/ou à mémoire de forme actuellement utilisés dans le domaine biomédical sont du type titane-nickel.

Néanmoins, on sait que le nickel est allergisant pour l'organisme et peut provoquer des réactions inflammatoires, malgré l'intérêt qu'il présente pour ses propriétés mécaniques, notamment de super élasticité et/ou de mémoire de forme. Par ailleurs, les alliages Ti-Ni possèdent une usinabilité médiocre qui engendre des ruptures prématurées des limes endodontiques (voir par exemple : Oiknine M., Benizri J., REV. ODONT. STOMATO. 36 (2007) 109-123) et sont parfois difficile à mettre en forme à froid.

Ces alliages connus présentent un caractère super élastique par la déstabilisation sous contrainte de la phase beta mère (cubique) par transformation en une phase martensitique alpha" (orthorhombique) réversible (Kim H.Y., Ikehara Y., et al, ACTA MATERIALIA 54 (2006) 2419-2429).

En outre, on connait des alliages à base de titane sans nickel (appelés « Gum Metals », Saito T., Furuta T. et al, SCIENCE 300 (2003) 464-467) qui sont considérés comme étant super élastiques car, bien que ne présentant pas de transformation martensitique sous contraintes, ils possèdent une faible rigidité et une élasticité recouvrable très importante.

Par ailleurs, on connait par le brevet français 2 848 810, par la demande de brevet US 2007/0137742 et par la demande de brevet WO 2005/093109 des alliages à base de titane et sans nickel.

La demande de brevet EP0449793 A1 divulgue des produits en alliage de titane pourvus d'une couche superficielle nitrurée et le procédé de leur fabrication.

Néanmoins, les alliages proposés dans cet art antérieur ne répondent pas de façon globalement satisfaisante à l'ensemble des critères requis, à la fois sur le plan mécanique et de la biocompatibilité notamment en surface. Par exemple, au regard de la compatibilité biologique, le brevet français ci-dessus propose un traitement de surface de l'alliage par dépôt de nitrure, et ce par une technique à base de plasma.

Cependant, cette technique connue n'est pas satisfaisante. En effet, le dépôt à l'aide de plasma ne permet pas un dépôt d'une couche de nitrure uniforme. Ceci a des conséquences dommageables ou gênantes dans le cas de dispositifs ou d'éléments présentant des formes particulières ou des parties ou zones difficilement accessibles (telles que des concavités ou similaires).

En outre, ledit brevet français décrit un procédé qui ne s'applique pas à un alliage à mémoire de forme et/ou super élastique.

Si la plupart des alliages sont donc à base de titane et de nickel, il a été proposé récemment des alliages super élastiques à base de titane sans nickel et particulièrement bien déformable à froid. L'article publié dans la revue JOURNAL OF THE MECHANICAL BEHAVIOR OF BIOMEDICAL MATERIALS 3 (2010) 559-564, par Bertrand E., Gloriant T. et al « Synthesis and characterisation of a new superelastic Ti-25Ta-25Nb biomedical alloy » montre de tels alliages à base de titane sans nickel.

Ainsi, le procédé de l'invention permet de résoudre les problèmes de l'art antérieur, en proposant de réaliser un alliage à base de titane, pour des applications biomédicales, à propriétés super élastiques et/ou à mémoire de forme, traité en surface, et qui répond à l'ensemble des conditions requises sur le plan mécanique, exprimées ci-dessus, et en outre qui est amélioré par rapport à l'art antérieur, au regard de la dureté superficielle, de la facilité d'usinage et du travail à froid, également de résistance à la stérilisation, tout en étant en outre parfaitement biocompatible.

L'invention est définie dans les revendications.

A cette fin, selon l'invention, le procédé de fabrication d'un alliage à base de titane, ne contenant pas de nickel, à propriétés super élastique et/ ou à mémoire de forme pour application biomédicale, du type comportant les phases successives de :
- préparation par fusion sous vide des différents métaux constitutifs de l'alliage souhaité, pour réaliser un lingot;
- optionnellement, homogénéisation sous vide du lingot par recuit à une première température, notamment supérieure à 900°C, consista nt en une montée en température du lingot, d'un maintien à cette température pendant une durée permettant l'homogénéisation complète
- première trempe ;
- mise en forme mécanique à température ambiante, telle que par laminage, tréfilage, usinage, ou équivalent ;
- traitement thermique de remise en solution en phase beta au delà du transus beta consistant en une montée en température jusqu'à une seconde température désirée, d'un maintien à cette température pour une certaine durée
- seconde trempe ;
caractérisé en ce que ladite phase de traitement thermique est réalisée sous atmosphère gazeuse, et constitue également une phase dite de nitruration, réalisant un traitement de surface par réaction avec ledit gaz, de manière à former de manière homogène en surface, une couche de nitrure, carbonitrure, oxynitrure.

Le lingot obtenu en première étape présente une masse variant de quelques dizaines à quelques centaines de grammes permettant de réaliser le dispositif biomédical.

Avantageusement, ledit gaz est de l'azote.

Ainsi, le procédé de l'invention introduit une étape de traitement de surface (par nitruration en phase gazeuse) en vue d'améliorer les propriétés mécaniques et de biocompatibilité superficielle de l'alliage.

Lors de l'étape de nitruration, on a veillé à un installer le lingot mis en forme dans une enceinte et tel que son agencement permette le dépôt de la couche en phase gazeuse sur la totalité de sa surface, y compris dans les concavités du lingot mis en forme. Pour ce faire, le lingot est suspendu par une chainette au coeur de l'enceinte constituant le four.

Les première et seconde trempes ont pour objectif de retenir la phase beta à température ambiante, pour obtenir l'effet super élastique désiré. On réalise, selon la composition de l'alliage, soit une trempe à l'eau, soit une trempe à l'air.

La phase d'homogénéisation est optionnelle en ce sens que, pour certaines compositions d'alliages, la phase de fusion peut conduire directement à l'obtention d'un lingot homogène. La phase d'homogénéisation est réalisée à une température supérieure à 900 °C. Par ailleurs, on sait que la température du « transus béta » est la température la plus basse à laquelle une phase 100 % « béta » de l'alliage puisse exister. Elle varie entre 600° C et 1050° C, en fonction de la composition de l'alliage.

De préférence, la phase simultanée de remise en solution en phase beta et de nitruration (dans le cas où le gaz est de l'azote) est réalisée à une température comprise entre 600 et 1050 °C, de préférence entre 800°C et 1050°C, sous une atmosphère gazeuse, de préférence d'azote, pendant une durée de quelques heures, pour obtenir une couche superficielle de nitrure variant de quelques microns à quelques dizaines de microns d'épaisseur, selon l'usage envisagé.

La durée de maintien à la température de recuit de la phase d'homogénéisation est comprise entre 12 heures et 20 heures, et de préférence de l'ordre de 16 heures.

En variante, ne faisant pas partie de la présente invention, la couche de nitrure (ou tout autre type de couche) peut être réalisée par une technique du type :
- plasma
- implantation ionique
- arc cathodique
- laser
- tout procédé PVD ou CVD.

La technique employée ne doit pas modifier la microstructure beta métastable de l'alliage qui est à l'origine de l'effet super élastique. Avantageusement, la phase de nitruration est conjuguée avec une phase simultanée de recristallisation et conduit à l'obtention d'une microstructure beta recristallisée.

La présente demande divulgue également d'une part un alliage tel qu'obtenu par la procédé ci-dessus, et d'autre part un dispositif à usage biomédical, incorporant ledit alliage.

Selon une forme préférée, l'alliage de l'invention comporte selon sa composition chimique en pourcentage atomique :
- Titane : entre 30 % et 98 %
- Niobium : entre 0 % et 40 %
- Molybdène : entre 0 % et 15 %
- Chrome : entre 0 % et 15 %
- Fer : entre 0 % et 15 %
- Zirconium : entre 0 % et 40 %
- Hafnium : entre 0% et 40 %
- Tantale : entre 0 % et 60 %
- Oxygène : entre 0 % et 2 %
- Azote : entre 0 % et 2 %
- Silicium : entre 0 % et 2 %
- Bore ; entre 0 % et 2 %
- Carbone : entre 0 % et 2 %
- Vanadium : entre 0 % et 15 %
- Tungstène : entre 0 % et 20 %
- Aluminium entre 0 % et 10 %
- Etain entre 0 % et 10 %
- Gallium : entre 0 % et 10 %

On mentionne ci-dessous quelques compositions de l'alliage de l'invention, données en pourcentage atomique :
Ti₇₄ Nb₂₆
Ti₇₂ Nb₁₈ Ta₁₀
Ti₇₄ Nb₂₀ Zr₆
Ti₇₆ Nb₂₃ N
Ti_{78.5} Nb₁₅ Zr_{2.5} Sn₄
Ti_{73.1} Nb₂₃ Ta_{0.7} Zr₂ O_{1.2}

Ainsi, contrairement à l'art antérieur, le procédé de l'invention s'applique à un alliage qui ne comporte pas de nickel.

L'invention sera bien comprise à la lumière de la description qui suit d'exemples illustratifs mais non limitatifs sur la base des dessins annexés dans lesquels :
- La figure 1 est une représentation graphique de la variation de la température en fonction du temps à laquelle est soumis le lingot d'alliage, selon les différentes étapes du procédé de l'invention.
- La figure 2 est une micrographie obtenue par microscope optique, montrant la microstructure beta métastable d'un exemple d'alliage Ti-25Ta-25Nb à coeur.
- La figure 3 montre une coupe transversale, observée par microscopie optique, de la microstructure de la surface nitrurée de l'alliage de la figure 2.
- La figure 4 montre une courbe de traction uni-axiale cyclée (charge/décharge successifs) montrant le caractère super élastique de l'alliage nitruré par la formation d'hystérésis entre charge et décharge due à la transformation martensitique alpha" sous contrainte.
- Les figures 5A et 5B montrent des micrographies obtenues par microscopie optique respectives d'un échantillon non nitruré (art antérieur) et nitruré (selon l'invention) après test de rayure par tribomètre pion-disque à charge et à nombres de cycles équivalents.

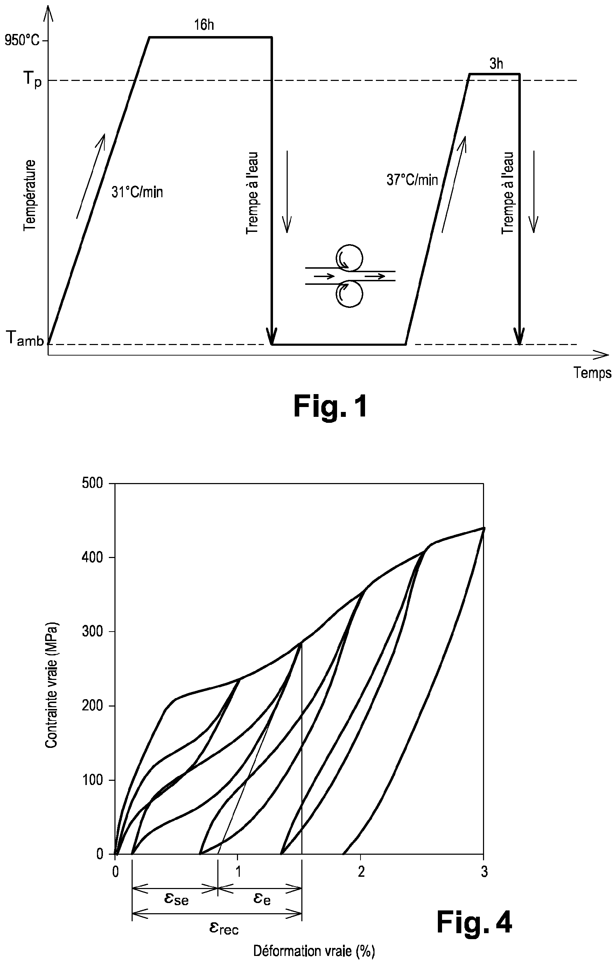

L'invention est maintenant décrite en regard de la figure 1 qui montre une courbe schématique des variations de température d'un lingot incluant des différents composés, et destiné à constituer l'alliage, en fonction du temps, au regard des étapes successives du procédé de l'invention.

Dans une étape préliminaire (non représentée sur la figure 1), on rassemble les différentes quantités de métaux qui rentreront dans la composition de l'alliage à réaliser, dans des proportions définies ci après. Le mélange de métaux est soumis à une opération de fusion préliminaire à une température comprise entre 2000 °C et 3000 °C. Avantageusement, cette étape préliminaire de fusion est réalisée en creuset froid par semi lévitation magnétique à l'aide d'un générateur à induction haute fréquence. On peut également utiliser des techniques de frittage, conventionnel ou rapide (flash).

Il importe, lors de cette étape préliminaire, de s'assurer de réaliser un mélange homogène, sans aucune inclusion ni contamination ou pollution d'éléments étrangers. A cet égard, la fusion est réalisée de préférence sous vide ou sous atmosphère contrôlée avec un gaz neutre (tel que de l'argon par exemple).

Les différents éléments susceptibles d'entrer dans la composition du lingot et donc du futur alliage incluent :
- le titane, pour une grande partie ou une partie majoritaire
- d'autres métaux : tantale, niobium, molybdène, zirconium, hafnium, vanadium, fer, chrome, tungstène qui constituent des éléments dits « beta-stabilisants », et éventuellement
- encore d'autres éléments tels que : aluminium, silicium, bore, carbone, oxygène, azote, étain, gallium... qui, ajoutés en faible quantité, sont susceptibles d'améliorer les propriétés de super élasticité.

Les différents composants sont choisis en termes qualitatif et quantitatif en vue de réaliser un alliage du type beta métastable par trempe, et permettant la formation d'une phase martensitique alpha", qui soit réversible, et procurant ainsi des propriétés super élastiques et/ou à mémoire de forme.

Le caractère « beta-métastable » de l'alliage se traduit par un faible module d'élasticité apparent variant de 10 GPa à 70 GPa se rapprochant de celui de l'os.

A partir du lingot fusionné, obtenu par l'étape préliminaire de fusion mentionnée ci-dessus, la seconde étape consiste en un « recuit d'homogénéisation » effectuée à haute température (typiquement entre 900 °C et 1200 °C) sous ultra vide.

A noter que sur le graphique de la figure 1, l'échelle de temps (abscisse) n'est pas respectée, pour des raisons pratiques.

Le lingot est maintenu à la température dite de recuit pendant une durée qui peut être de plusieurs heures. La température et la durée du traitement dépendent de l'alliage considéré. Au final, un alliage complètement homogène doit être obtenu.

La phase de recuit d'homogénéisation s'achève par une première trempe, de préférence par une trempe à l'eau, de manière à retenir une microstructure « beta » métastable à la température ambiante qui favorisera les opérations de mise en forme. La trempe est réalisée en une fraction de seconde par chute du lingot depuis la température de recuit dans un bac d'eau à température ambiante.

Ensuite, on réalise les opérations de mise en forme et d'usinage, à froid, c'est-à-dire à température ambiante. Cette étape vise à conformer le lingot selon la forme du dispositif ou élément souhaité, pour une application biomédicale telle que mentionnée ci-dessus. L'opération de mise en forme est adaptée bien entendu à la configuration et la forme du produit à réaliser et fait appel à des techniques connues d'usinage, ou de mise en forme telle que tréfilage, laminage, extrusion ou autre.

Le dispositif biomédical ainsi fabriqué, constitué de l'alliage souhaité, est alors soumis à un traitement thermique de remise en solution au dessus de la température T_{β} qui est la température de transus beta typiquement entre 600 °C et 1050 °C.

Le dispositif est maintenu à cette température pendant une durée de quelques dizaines de minutes à quelques heures, et ce, sous atmosphère gazeuse tel que de l'azote par exemple. L'appareil utilisé est un four connu en lui-même.

Ce traitement mené à température constante, vise deux objectifs :
- l'obtention d'une microstructure « beta recristallisée » présentant une taille de grain affinée afin d'améliorer et optimiser les propriétés mécaniques du dispositif biomédical final ;
- le dépôt, lors de son séjour dans le four, d'une couche de nitrure en surface sur le dispositif, par la réaction directe à chaud, entre l'alliage constitutif du dispositif, et l'azote gazeux introduit dans le four de trempe. Ce traitement constitue un processus de nitruration en phase gazeuse. La durée de cette étape de nitruration varie entre 0, 5 et 10 heures en fonction des compositions de l'alliage, des épaisseurs souhaitées et de la forme du dispositif. La température maintenue durant cette étape de nitruration est comprise entre 600 et 1050°C.

Enfin, à l'issu de la phase de nitruration / recristallisation, on réalise une seconde trempe, de préférence une trempe à l'eau, qui ramène la température du dispositif à la température ambiante. Cette seconde trempe permet de maintenir la microstructure beta de l'alliage sous une forme métastable.

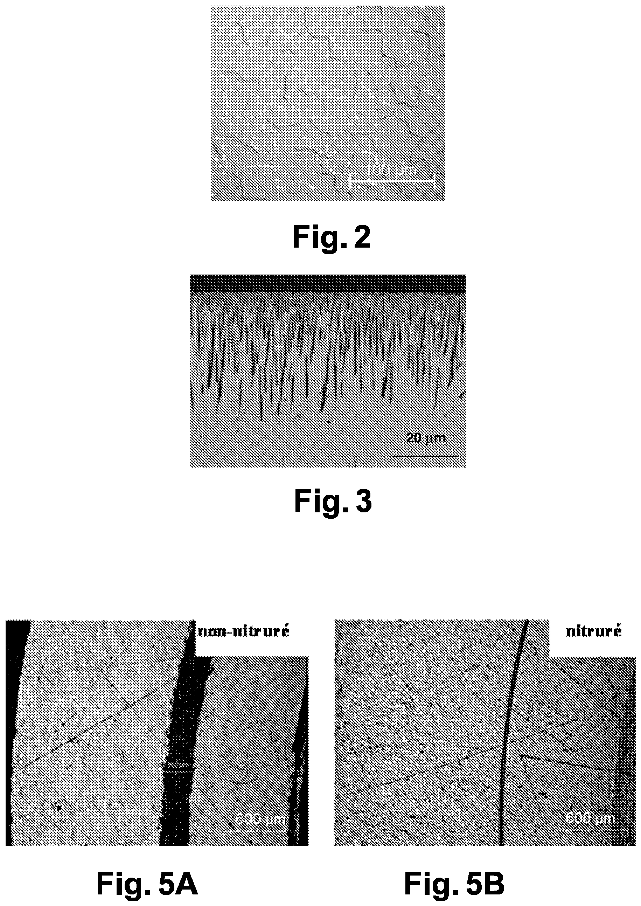

Le demandeur a réalisé des essais en laboratoire sur des échantillons d'alliage réalisé selon le procédé de l'invention décrit ci-dessus. L'alliage en question est super élastique du type beta-métastable recristallisé, avec une taille de grain comprise entre 10 et 60 microns (voir la microstructure beta du coeur de l'alliage en figure 2). Sa composition, exprimée en pourcentage massique est : Ti (50%), Ta (25%) et Nb (25%). La phase de recristallisation-nitruration avait été effectuée à 800°C pendant une durée de 3 heures et a conduit à une couche de nitrure de titane de quelques dizaines de microns d'épaisseur. La microstructure du nitrure en surface est montrée en coupe transversale sur la figure 3, où les parties sombres correspondent à la zone nitrurée constituée d'aiguilles riches en azote (nitruration interne).

La couche de nitrure de faible épaisseur n'altère pas les propriétés super élastiques de l'alliage ainsi obtenu. La figure 4 montre la courbe de traction cyclée concernant l'alliage nitruré qui montre la présence d'hystérésis charge/décharge caractéristiques de l'effet super élastique. L'essai de traction cyclé présenté sur cette figure a été mené sur une éprouvette plate d'un millimètre d'épaisseur obtenu par le présent procédé de fabrication. Les cycles charge/décharge ont été effectués avec une incrémentation de 0.5% en déformation.

Un alliage nitruré réalisé par le procédé de l'invention, présente par rapport à un alliage de même composition, non nitruré, une augmentation de la dureté superficielle (mesuré 4 fois supérieure par micro dureté Vickers), ce qui entraine une très forte augmentation de la résistance à l'usure (volume usé réduit de 85%) ainsi qu'une nette diminution du coefficient de friction (divisée par 5). Les figures 5A et 5B montrent chacune une vue de dessus d'un échantillon d'alliage ayant subi un test de rayure, respectivement non nitruré (5A - art antérieur) et nitruré (5B - selon l'invention). Les sillons (de couleur foncée) sont obtenus sous charge de 25g après 200 cycles en rotation à l'aide d'un tribomètre pion-disque. On constate que l'alliage selon l'invention (figure 5B) présente une résistance bien supérieure en surface.

La phase de nitruration réalisée en phase gazeuse comme décrit ci-dessus présente de nombreux avantages par rapport aux techniques de nitruration connues.
- Le dépôt d'une couche de nitrure d'épaisseur sensiblement homogène, même sur des objets de forme complexe, comme le sont d'ailleurs dans la plupart des cas les dispositifs à usage biomédical ;
- Grande simplicité de mise en oeuvre ;
- La nitruration (c'est-à-dire le dépôt d'un nitrure) est réalisée de manière simultanée à la recristallisation de l'alliage lors de la mise en solution ; ceci n'est pas possible par les autres méthodes de nitruration qui suppose de n'effectuer celles-ci qu'après recristallisation sous vide.
- Une très bonne adhérence de la couche sur l'alliage par formation de nitruration interne près de la surface.

De plus, la seconde trempe du procédé de l'invention, qui suit l'étape simultanée de nitruration/recristallisation, présente l'avantage de maintenir la microstructure beta métastable au coeur de l'alliage, pour obtenir l'effet super élastique.

L'invention n'est pas limitée au dépôt de nitrure, mais inclut également le dépôt de couche superficielle d'oxynitrure, ou de carbonitrure. Dans ce cas, on utilise un gaz ou un mélange de gaz adapté, en l'occurrenceen ajoutant du dioxyde de carbone, du monoxyde d'azote ou même de l'air pour l'obtention d'oxynitrure, ou de carbonitrure en surface.

L'alliage réalisée selon le procédé de l'invention, et selon les compositions indiquées, présente des avantages suivant par rapport aux alliages du type titane/nickel et notamment une capacité à la déformation à froid très importante, et aptitude à l'usinage supérieure, ces deux avantages étant particulièrement appréciés dans le cas de dispositifs à application biomédicale. A titre d'exemple, les limes endodontiques présentent dans l'art antérieur une usure relativement importante et des risques de fractures non négligeables causées par les stries d'usinage.

En outre, la capacité de coupe de l'alliage selon l'invention est plus importante que celle des alliages de l'art antérieur. La couche de nitrure améliore les propriétés de dureté et de résistance à l'usure, tout en procurant des effets bénéfiques sur le plan de la biocompatibilité. Dans les dispositifs d'application cardio-vasculaires, tels que des stents, on observe, pour ce qui est de l'alliage de l'invention, une meilleure hémocompatibilité. Enfin, le dispositif biomédical ainsi réalisé résiste mieux aux opérations de stérilisation et est moins sensible à l'activité bactériologique grâce à la présence de la couche de nitruration.

En résumé, le procédé selon l'invention permet de fabriquer un alliage à base de titane pour application biomédicale, qui présente des propriétés super élastiques et/ou à mémoire de forme, avec tous les avantages qui en découlent et exprimés ci-dessus, et en outre ces propriétés étant pour certaines d'entre elles renforcées par la couche de nitrure, qui elle-même apporte d'autres capacités ou propriétés mécaniques, et enfin cette dernière renforce ou améliore la biocompatibilité du dispositif à usage biomédical.

## Revendications

1. Procédé de fabrication d'un alliage à base de titane, ne contenant pas de nickel, à propriétés super élastique pour application biomédicale, du type comportant les phases successives de :
- préparation par fusion sous vide des différents métaux constitutifs de l'alliage souhaité, pour réaliser un lingot ;
- première trempe ;
- mise en forme mécanique à température ambiante, telle que par laminage, tréfilage, usinage, ou équivalent ;
- traitement thermique de remise en solution en phase beta au delà du transus beta consistant en une montée en température jusqu'à une seconde température désirée, d'un maintien à cette température pour une certaine durée ;
- seconde trempe ;
**caractérisé en ce que** ladite phase de traitement thermique est réalisée sous atmosphère gazeuse, et constitue également une phase dite de nitruration, réalisant un traitement de surface par réaction avec ledit gaz, de manière à former de manière homogène en surface, une couche de nitrure, carbonitrure, oxynitrure, et **en ce que** l'alliage est en phase beta méta stable à la température ambiante après la trempe.

2. Procédé selon la revendication 1, **caractérisé en ce que** la phase de nitruration est réalisée à la seconde température comprise entre 600° C et 1050°C, de préférence entre 800° C et 1050° C.

3. Procédé selon la revendication 1 ou 2, **caractérisé en ce que** la phase de nitruration est réalisée sous atmosphère d'azote.

4. Procédé selon l'une des revendications 1 à 3, **caractérisé en ce que** la phase de nitruration est conjuguée avec une phase simultanée de recristallisation.

5. Procédé selon l'une des revendications 1 à 4, **caractérisé en ce que** la première et la seconde trempes sont réalisées sous forme de trempes à l'eau ou à l'air.

## Patentansprüche

1. Verfahren zur Herstellung einer Legierung auf Titanbasis, die kein Nickel enthält, mit superelastischen Eigenschaften für biomedizinische Anwendung, vom Typ, der die folgenden sukzessiven Phasen umfasst:
- Vorbereiten durch Schmelzen unter Vakuum der unterschiedlichen Metalle, die die gewünschte Legierung bilden, um einen Barren auszubilden;
- erstes Härten;
- mechanisches Formen bei Umgebungstemperatur, wie durch Walzen, Ziehen, Bearbeiten oder Gleichwertiges;
- Wärmebehandeln zur Wiederauflösung in Beta-Phase über den Transus-Beta hinaus, das aus einem Temperaturanstieg bis zu einer zweiten gewünschten Temperatur, einem Halten bei dieser Temperatur während einer bestimmten Dauer besteht;
- zweites Härten;
**dadurch gekennzeichnet, dass** die Wärmebehandlungsphase unter Gasatmosphäre ausgeführt wird und auch eine sogenannte Nitrierungsphase bildet, die eine Oberflächenbehandlung durch Reaktion mit dem Gas ausführt, so dass auf homogene Art auf der Oberfläche eine Nitrid-, Carbonitrid-, Oxinitridschicht gebildet wird, und dadurch, dass die Legierung in Beta-Phase bei der Umgebungstemperatur nach dem Härten metastabil ist.

2. Verfahren nach Anspruch 1, **dadurch gekennzeichnet, dass** die Nitrierungsphase bei der zweiten Temperatur, die zwischen 600 °C und 1050 °C, bevorzugt zwischen 800 °C und 1050 °C liegt, ausgeführt wird.

3. Verfahren nach Anspruch 1 oder 2, **dadurch gekennzeichnet, dass** die Nitrierungsphase unter Stickstoffatmosphäre ausgeführt wird.

4. Verfahren nach einem der Ansprüche 1 bis 3, **dadurch gekennzeichnet, dass** die Nitrierungsphase mit einer gleichzeitigen Rekristallisationsphase konjugiert ist.

5. Verfahren nach einem der Ansprüche 1 bis 4, **dadurch gekennzeichnet, dass** das erste und das zweite Härten in Form von Härten mit Wasser oder mit Luft ausgeführt werden.

## Claims

1. Method for manufacturing a titanium-based alloy, not containing nickel, with superelastic properties for biomedical application, of the type including the successive phases of:
- preparation by vacuum melting of the various constituent metals of the alloy sought, to produce an ingot;
- first quenching;
- mechanical forming at ambient temperature, such as by rolling, wire drawing, machining, or equivalent;
- solution heat treatment in the beta phase beyond the beta transus consisting of a temperature rise to a sought second temperature, holding at this temperature for a certain time;
- second quenching;
**characterised in that** said heat treatment phase is carried out in a gaseous atmosphere, and also represents a so-called nitriding phase, carrying out a surface treatment by reaction with said gas, so as to form homogeneously on the surface, a layer of nitride, carbonitride, oxynitride, and **in that** the alloy is in the beta phase metastable at the ambient temperature after quenching.

2. Method according to claim 1, **characterised in that** the nitriding phase is carried out at the second temperature between 600°C and 1050°, preferably between 800°C and 1050°C.

3. Method according to claim 1 or 2, **characterised in that** the nitriding phase is carried out in a nitrogen atmosphere.

4. Method according to one of claims 1 to 3, **characterised in that** the nitriding phase is combined with a simultaneous recrystallisation phase.

5. Method according to one of claims 1 to 4, **characterised in that** the first and second quenching operations are carried out in the form of water or air quenching.
